# EUROPEAN PATENT APPLICATION

(11) **EP 4 570 915 A1**
(43) Date of publication of application: **18.06.2025**
(21) Application number: 23215454.2
(22) Date of filing: 11.12.2023
(51) Int. Cl.: C12Q 1/00, A61B 5/00, C07K 14/47, C12Q 1/04, C12Q 1/34, G01N 33/543, G01N 33/573

(54) **DEVICE AND METHOD FOR DETECTING ENZYMATIC ACTIVITY**

(71) Applicant: Stichting IMEC Nederland, 6708 WH Wageningen (NL)
(72) Inventor: Tibbe, Arjan, 7425 PG Deventer (NL); Armstrong, Rachel, 5616 HB Eindhoven (NL)
(74) Representative: Körfer, Thomas

(57) **Abstract**

A device (100) is provided for detecting enzymatic activity in mucus layer. The device comprises at least one mucin molecule (103) corresponding to an enzyme of a first type, wherein the mucin molecule (103) is coupled to a surface (102) of the device and is configured to be in contact with mucus layer contents, at least one probe (104) coupled to an end of the mucin molecule (103) opposite to the surface (102) of the device, and a detector (101) configured to detect a presence of the probe (104) and/or a property of the probe (104) in order to detect a status of the mucin molecule (103).

## Description

The invention relates to the detection of enzymatic activity in the mucus layer that lines the walls of the body's organs and especially the mucus layer of the gastrointestinal (GI) tract, the respiratory tract and the reproductive organs.

The mucus layer may be regarded as the first line of defense against the infiltration of microorganisms. A healthy and intact mucus layer is essential to prevent colonization and invasion by pathogens. A healthy intact mucus layer contains a healthy population of commensal bacteria that maintain a symbiotic relationship with the host. By measuring the presence of enzymes produced by the commensal mucus residing bacteria, the type of bacteria and, thereby, the health status of the mucus layer may be identified.

At present, protease activity detection schemes generally relate to ex *situ* measurements performed on a cultured molecule in a controlled environment. For example, the document Ji Ji et al., Electrochemical detection of the activities of thrombin and its inhibitor, Electrochemistry Communications, Volume 16, Issue 1, 2012, Pages 53-56, doi: 10.1016/j.elecom.2012.01.004 discloses the detection of thrombin activities in a controlled environment. However, such a detection scheme cannot work when measuring biomarkers in complex matrices like the GI-tract.

Accordingly, an object of the invention is to provide a device and a method to facilitate *in vivo* measurements, especially within the mucus layer of the GI-tract, to measure the presence of enzymes produced by commensal bacteria residing in the mucus layer.

The object is solved by the features of the first independent claim for the device and by the features of the second independent claim for the method. The dependent claims contain further developments.

According to a first aspect of the invention, a device is provided for detecting enzymatic activity in the mucus layer. The device comprises at least one mucin molecule corresponding to an enzyme of a first type, wherein the mucin molecule is coupled to a surface of the device and is configured to be in contact with mucus layer contents, at least one probe coupled to an end of the mucin molecule opposite to the surface of the device, and a detector configured to detect a presence of the probe and/or a property of the probe in order to detect a status of the mucin molecule.

The device may be an ingestible device, where the detector or sensor may be arranged in an ingestible structure or packaging, such as an ingestible sensing pill. Alternatively, the device may be an implantable device, or a device that can be in physical contact with the mucus layer contents.

For the ingestible device, the enzyme-specific mucin molecule may be coupled to a surface of the ingestible structure, especially to an exterior surface of the ingestible structure, so that the mucin molecule is in contact with the contents of the mucus layer, i.e. the sample matrix, especially within the GI-tract, whereby the probe may be coupled to the end of the mucin molecule, e.g., to the top side, opposite to the surface of the ingestible structure.

Accordingly, the detector or sensor may detect or sense a presence of the probe in order to detect a status of the mucin molecule. Additionally or alternatively, the detector may detect or sense or measure a property of the probe in order to detect a status of the mucin molecule.

The status of the mucin molecule according to the present invention is defined herein as its glycosylation content and/or extent of glycosylation and/or alterations in glycosylation pattern and/or degradation and/or removal, and/or presence or absence.

Enzymes produced by commensal microbes residing in the mucus layer, including but not limited to glycosidases, sulphatases, or sialidases, are able to digest glycan structures of the mucin molecule. Degradation and/or removal and/or alteration of the glycosylation and/or glycosylation pattern releases the mucin molecule backbone and renders it accessible to proteases, which in turn digest (degrade and/or remove) the mucin molecule. Degradation and/or removal of the mucin molecule releases (removes) the probe from the sensor surface and results in a detectable alteration of the signal and/or signal intensity level.

Advantageously, the alteration of the signal and/or signal intensity level is detected. Preferably, the alteration of the signal and/or signal intensity level is a disappearance of the signal and/or a decrease of the signal intensity level.

In case the enzyme for digesting the glycan structure is not present, the mucin molecule backbone remains protected from the proteases and the probe remains attached to the mucin molecule and, thereby, to the sensor surface. In this case, the signal and/or signal intensity remains at the unchanged, i.e. at the starting level.

Thus, advantageously, the presence of enzymes produced by the commensal mucus residing bacteria can be detected so that the type of bacteria and, thereby, the health status of the mucus layer can be identified.

A healthy and intact mucus layer contains a healthy population of commensal bacteria maintaining a symbiotic relationship with the host. The mucus layer serves as a carbon and energy source for mucus residing commensal bacteria, predominantly in the form of glycans. The mucus layer residing commensal bacteria produce mucus-degrading enzymes such as glycosidase, sulphatase, and sialidase cleaving the mucus network to enhance utilization as an energy source to produce monosaccharides utilized by the mucus residing bacteria. In turn, the mucus residing commensal bacterial produce short-chain fatty acids to maintain a functional mucus layer barrier.

The mucus layer is composed of mucins consisting of a large family of heavily O-glycosylated proteins. Mucins are primarily defined by their variable tandem repeat (TR) domains that are densely decorated with different O-glycan structures in different patterns.

Human TR-O-glycodomains consist of approximately 200 amino acids and are small parts of the mucins that can be produced by genetically engineered human HEK293 cells using the method according to Nason et al., Nat Commun 12, 4070 (2021), Display of the human mucinome with defined O-glycans by gene engineered cells.

Preferably, the mucin molecule comprises tandem repeat (TR) domains that defines O-glycan binding sites. For instance, the TR sequence defines the density of O-glycans. The O-glycan structures can be composed of natural, engineered or modified sugar groups hereby creating enzymatic selectivity or blocking enzymatic digesting.

Preferably, the mucin molecule is configured to be digested by the enzyme of the first type, especially produced by the commensal microbes that reside in the mucus layer. For instance, the mucin molecule may be configured to be digested exclusively by the enzyme of the first type. In this regard, the O-glycan groups that cover the mucin backbone (amino acid backbone) may be configured to be digested exclusively by the enzyme of the first type.

Alternatively, the O-glycan groups may be configured to be digested by a combination of enzymes wherein the enzyme of the first type may digest the first sugar group of the glycan domain and the next available sugar group may be digested by an enzyme of a second type. Advantageously, the activity of a specific enzyme or a combination of enzymes produced by the commensal bacteria residing in the mucus layer can be detected.

Preferably, the probe is an optical probe and the detector is configured to detect an optical signal from the probe. In a preferred embodiment, the optical probe is a fluorescent probe and the detector is configured to detect fluorescence of the fluorescent probe, especially as the property of the probe. In another preferred embodiment, the optical probe may be an optical absorption probe.

In a further preferred embodiment, the probe is a redox probe and the detector is configured to detect an oxidation-reduction activity of the redox probe, especially as the property of the probe. For instance, the detector may detect an oxidation-reduction potential of the redox probe.

Advantageously, a fast detection time and a high detection sensitivity is achieved.

Preferably, the device comprises a linker molecule configured to couple the mucin molecule to the surface of the device, the linker molecule being inert to the mucus layer contents, especially to the enzymes produced by the commensal microbes that reside in the mucus layer.

For example, in the case of an ingestible device, the linker molecule may be inert to the enzymes present in the GI-tract. For instance, the enzymatic activity may be monitored by measuring the enzymatic digestion of the mucin molecule. The enzymatic activity may lead to a decrease of the number sensor probes and as such loss of signal.

Advantageously, nonspecific binding is not measured by the sensor. Thereby, false positive enzymatic protein digestion registration can be prevented and the detection accuracy can be enhanced.

The linker molecule may be any linker molecule used in the art, such as synthetic amino acid linkers, disulfide linkers, hydrazone linkers. Additionally or alternatively, the linker molecule may comprise or be protein-substrate combinations such as streptavidin-biotin and avidin-biotin, silane molecules containing linkable functional groups like alkanes, primary amines, carboxylic acids, N-hydroxysuccinimde esters, azides, alkynes and so on.

Preferably, the device comprises at least one further mucin molecule corresponding to an enzyme of a second type, wherein the further mucin molecule is coupled to the surface of the device and is configured to be in contact with the mucus layer contents, especially in contact with enzymes produced by the commensal bacteria that reside in the mucus layer, and at least one further probe coupled to an end of the further mucin molecule opposite to the surface of the device.

In this regard, the detector is configured to detect a presence of the further probe and/or a property of the further probe in order to detect a status of the further mucin molecule. Advantageously, the presence of different enzymes produced by the commensal mucus residing bacteria can be detected.

Preferably, the further mucin molecule is configured to be digested by the enzyme of the second type, especially produced by commensal microbes that reside in the mucus layer. For instance, the further mucin molecule may be configured to be digested exclusively by the enzyme of the second type. In this regard, the O-glycan groups that cover the mucin backbone (amino acid backbone) may be configured to be digested exclusively by the enzyme of the second type.

Alternatively, the O-glycan groups may be configured to be digested by a combination of enzymes wherein the enzyme of the first type may digest the first sugar group of the glycan domain and the next available sugar group may be digested by an enzyme of a second type.

Advantageously, the activity of different enzymes in the mucus layer, especially of enzymes produced by the commensal bacteria residing the mucus layer, can be specifically detected, thereby obtaining an enzymatic signature, e.g., the distribution of enzymes along the mucus layer, e.g. within the GI-tract.

Preferably, the detector is configured to detect the presence of the probe and the presence of the further probe simultaneously. Additionally or alternatively, the detector is configured to detect the property of the probe and the property of the further probe simultaneously. Advantageously, a parallel detection of the enzymatic activity can be facilitated.

Preferably, the detector is configured to detect the presence of the probe and the presence of the further probe sequentially. Additionally or alternatively, the detector is configured to detect the property of the probe and the property of the further probe sequentially. Advantageously, the accuracy of the enzymatic activity detection can be further enhanced.

Preferably, the mucin molecule and the further mucin molecule are coupled to the surface of the device at predefined locations, especially in an array formation. Advantageously, the presence of different enzymes can be detected in a simplified manner based on the locations of the different mucin molecules in the array.

Preferably, the status of the mucin molecule or the further mucin molecule is the enzymatic degradation and/or removal of the mucin molecule or the further mucin molecule.

According to a second aspect of the invention, a method is provided for detecting enzymatic activity in mucus layer. The method comprises the steps of coupling at least one mucin molecule corresponding to an enzyme of a first type to a surface of a device such that the mucin molecule is in contact with mucus layer contents, coupling at least one probe to an end of the mucin molecule opposite to the surface of the device, and detecting a presence of the probe and/or a property of the probe in order to detect a status of the mucin molecule.

Preferably, the method further comprises the steps of coupling at least one further mucin molecule corresponding to an enzyme of a second type to the surface of the device such that the further mucin molecule is in contact with the mucus layer contents, coupling at least one further probe to an end of the further mucin molecule opposite to the surface of the device, and detecting a presence of the further probe and/or a property of the further probe in order to detect a status of the further mucin molecule.

Preferably, the method further comprises the step of detecting the presence of the probe and the presence of the further probe simultaneously. Additionally or alternatively, the method comprises the step of detecting the property of the probe and the property of the further probe simultaneously.

Preferably, the method further comprises the step of detecting the presence of the probe and the presence of the further probe sequentially. Additionally or alternatively, the method comprises the step of detecting the property of the probe and the property of the further probe sequentially.

Preferably, the status of the mucin molecule or the further mucin molecule is the enzymatic degradation and/or removal of the mucin molecule or the further mucin molecule.

It is to be noted that the method according to the second aspect corresponds to the device according to the first aspect and its implementation forms. Accordingly, the method of the second aspect may have corresponding implementation forms. Further, the method of the second aspect achieves the same advantages and effects as the device of the first aspect and its respective implementation forms.

Exemplary embodiments of the invention are now further explained with respect to the drawings by way of example only, and not for limitation. In the drawings:
- Fig. 1: shows a first exemplary embodiment of the device according to the first aspect of the invention;
- Fig. 2: shows an exemplary degradation process of the mucin molecule;
- Fig. 3: shows a first exemplary embodiment of the detector;
- Fig. 4: shows a second exemplary embodiment of the detector;
- Fig. 5: shows a second exemplary embodiment of the device according to the first aspect of the invention;
- Fig. 6: shows a third exemplary embodiment of the device according to the first aspect of the invention;
- Fig. 7: shows an exemplary enzymatic activity as a function of time; and
- Fig. 8: shows an exemplary embodiment of the method according to the second aspect of the invention.

Reference will now be made in detail to the embodiments of the present invention, examples of which are illustrated in the accompanying drawings. However, the following embodiments of the present invention may be variously modified and the range of the present invention is not limited by the following embodiments.

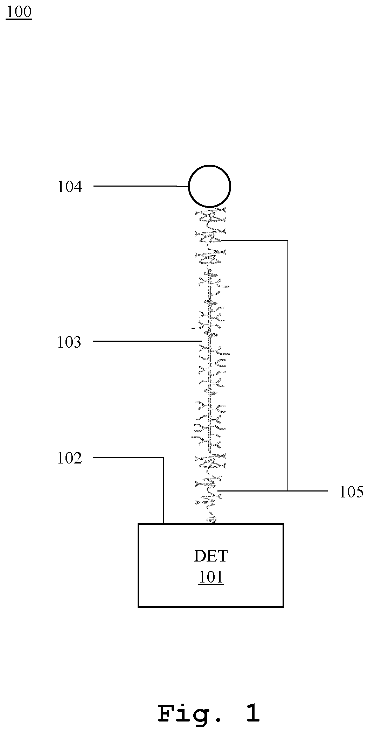

In Fig. 1, a first exemplary embodiment of the device 100 according to the first aspect of the invention is illustrated. Although the device 100 is exemplary illustrated as an ingestible device, the device 100 can also be an implantable device. The device 100 may comprise a detector or sensor 101 arranged in an ingestible structure, e.g., a pill, or an implantable structure, such that an external surface 102 of the structure, i.e., a detector or sensor surface, can be facilitated for the detection or sensing.

The device 100 may further comprise a mucin molecule 103 of a first type, especially with known amino acid sequence and structure. As such, the mucin molecule 103 may be configured to be digested primarily by an enzyme of a first type produced in the mucus layer. The mucin molecule 103 may be attached to the detector surface 102 so that the mucin molecule 103 is in contact with the enzymes produced by the commensal bacteria that reside in the mucus layer, e.g., are present in the GI-tract contents.

The device 100 may further comprise a probe or probe molecule 104 attached to an end of the mucin molecule 103, especially to the end of the mucin molecule 103 opposite to the detector surface 102.

In this regard, the mucin molecule 103 may be attached to the detector surface 102 through a linker molecule 105. Additionally, the probe 104 may be attached to the mucin molecule 103 in the similar manner, i.e., through a further linker molecule 105. The linker molecule 105 may be inert to the contents of the mucus layer, e.g., the contents of the GI-tract, especially to the enzymes produced in the GI-tract.

For example, the probe 104 may be a fluorescent probe and the detector 101 may measure a fluorescence of the probe 104. Alternatively, the probe 104 may be a redox probe and the detector 101 may measure an oxidation-reduction potential (ORP) of the probe 104. As such, a reduction in the measured fluorescence or in the measured ORP may indicate the status, in particular the degradation or removal of the mucin molecule 103, i.e., the mucin protein, thereby detecting an enzymatic activity in the mucus layer, e.g., within the GI-tract.

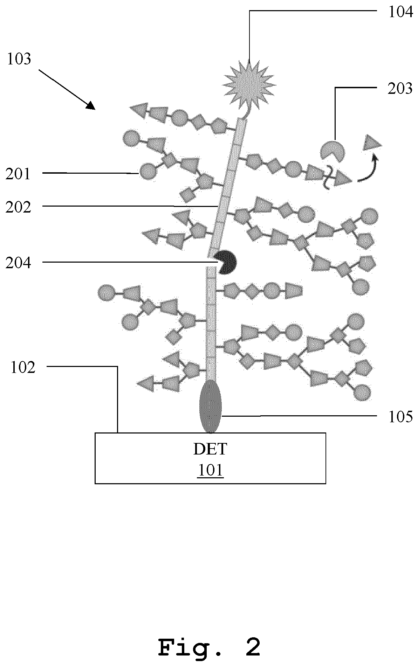

In Fig. 2, an exemplary degradation process of the mucin molecule 103 is illustrated.

Generally, the mucus barrier layer is composed of mucins consisting of a large family of heavily O-glycosylated proteins and constituting the major macromolecules in most body fluids. The mucins can be primarily defined by their variable tandem repeat (TR) domains that are densely decorated with different O-glycan structures in distinct patterns.

For example, human TR-O glycodomains consisting of approximately 200 amino acids may be small parts of the mucin molecules that can be produced by genetically engineered human HEK293 cells.

In particular, the mucus layer may serve as a carbon and energy source for mucus residing commensal bacteria, predominantly in the form of glycans. As an adaptation to residing in a glycan-rich environment, these bacteria produce mucus-degrading enzymes such as glycosidase, sulphatase, and sialidases, which can cleave the mucus network to enhance the utilization of mucus as an energy source. These bacterial species can cleave mucus O-glycans to produce monosaccharides utilized by the mucus-residing bacteria.

In return, these bacteria produce short-chain fatty acids (SCFAs) such as butyrate to regulate intestinal homeostasis. SCFAs may be regarded as an energy source for the epithelial cells and help maintain a functional barrier.

Generally, Inflammatory Bowel Disease (IBD) patients may display a reduction of commensal bacteria, including SCFA-producing bacteria, resulting in mucosal layer damage and leading to a dysfunctional barrier. This results in bacterial penetration, thereby triggering an inflammatory cascade against the invading bacteria. As such, by measuring the presence of enzymes produced by the commensal mucus-residing bacteria, the type of bacteria and the health status of the mucus layer can be identified.

Turning back to Fig. 2, the mucin molecule 103 may comprise O-glycans 201 covering the protein backbone 202. In the presence of the O-glycan-degrading enzymes 203, such as glycosidase, sulphatase, or sialidase, the O-glycan sugar groups 201 may be digested one by one by the O-glycan-degrading enzymes 203 and the protein backbone 202 may become exposed to proteases 204 that are present inside the sample matrix. In case the enzymes 203, 204 digest the mucin molecule 103,i.e. the status of the mucin molecule changes, the probe 104 may be released, especially from the detector surface 102, which may result in the disappearance and/or reduction of the measured fluorescence or of the measured ORP.

As such, the digestion process illustrated in Fig. 2 can be regarded as a two-step process:
1. The digestion of the O-glycans 201 of the mucin molecule 103 by the enzymes 203 produced by the commensal bacteria that reside in the mucus layer, and
2. After the removal of sufficient O-glycans, the amino acid backbone 202 of the mucin molecule 103 may become exposed to the "general available" proteases 204, which may result in a break, i.e. degradation, of the mucin molecule 103 and therefore a release (removal) of the probe 104.

It is to be noted that the linker molecule 105 may not be cleavable by the enzymes 203 and the proteases 204, especially to avoid false positive measurements of protein degradation. In a further aspect, the sample matrix may not contain enzymes capable of cleaving the linker molecule.

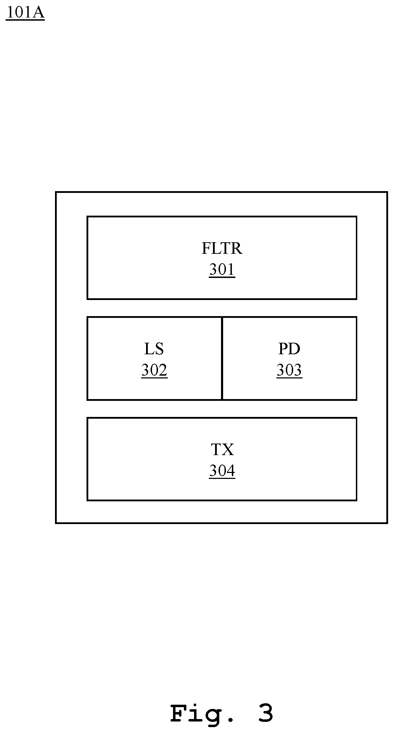

In Fig. 3, a first exemplary embodiment of the detector 101A is illustrated. The detector 101A may be a fluorescence detector and may detect the fluorescence of the probe 104.

For example, the detector 101A may comprise a filter or grating structure 301 configured for an excitation light signal of a predefined wavelength and further for an emission light signal of a predefined wavelength. For instance, the filter structure 301 may be arranged at or near the detector surface 102.

The detector 101A may further comprise a light source 302, such as a light-emitting diode, which may generate the excitation light signal to illuminate the probe 104 especially through the filter structure 301, e.g., to achieve the excited state of the probe molecule.

The detector 101A may further comprise a photodetector 303, such as a photodiode, which may receive the fluorescent emission or the emission light signal from the probe 104 or may detect the fluorescent emission or the emission light signal of the probe 104, especially through the filter structure 301. The fluorescent emission may result from the transition of the probe molecule from the excited state to the reference or ground state.

The detector 101A may further comprise a transmitter 304 that may receive the fluorescent measurements from the photodetector 303, e.g., the photodiode currents or voltages, and may transmit the measurements to an external device, especially wirelessly, for further pursing and/or processing of the measurement data.

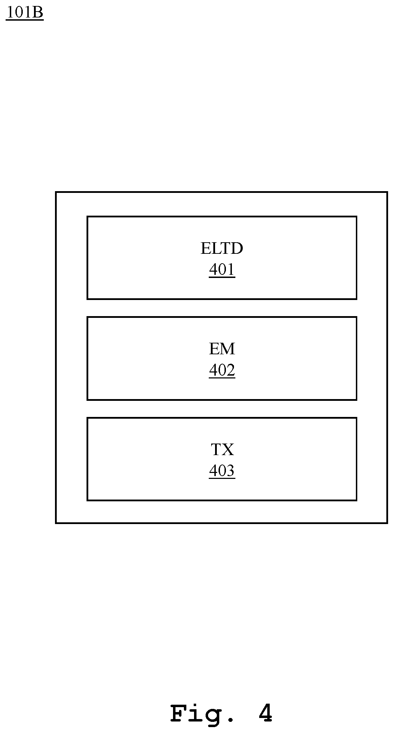

In Fig. 4, a second exemplary embodiment of the detector 101B is illustrated. The detector 101B may be an ORP detector and may detect or measure the ORP of the probe 104.

For example, the detector 101B may comprise an electrode 401 configured to be in electrically coupled to the probe 104. For instance, the electrode 401 may be arranged at or near the detector surface 102.

The detector 101B may further comprise an electrometer 402, such as a potentiometer, electrically coupled to the electrode 401. The electrometer 402 may measure an ORP of the probe 104.

The detector 101B may further comprise a transmitter 403 that may receive the ORP measurements from the electrometer 402 and may transmit the measurements to an external device, especially wirelessly, for further pursing and/or processing of the measurement data.

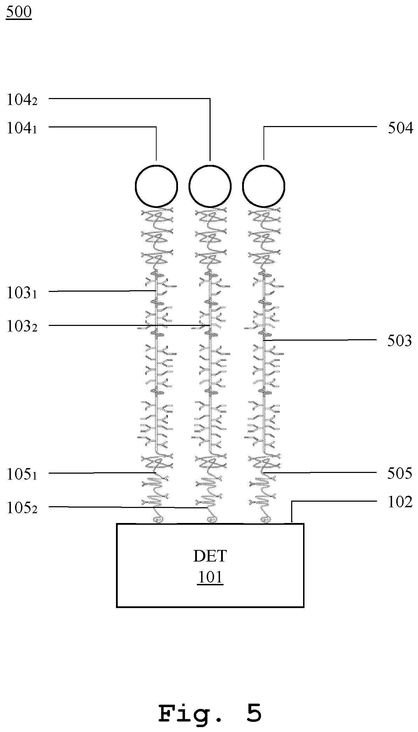

In Fig. 5, a second exemplary embodiment of the device 500 according to the first aspect of the invention is illustrated. The device 500 differs from the device 100 in that the device 500 comprises a plurality of mucin molecules of a first type, exemplarily two mucin molecules of the first type 103₁, 103₂, to form a cluster or array of mucin molecules of the first type.

In this regard, each of the mucin molecules 103₁, 103₂ may be configured to be digested primarily by the enzyme of the first type produced in the mucus layer. Furthermore, each of the mucin molecules 103₁, 103₂ may be attached to the detector surface 102 through a respective linker molecule 105₁, 105₂. Moreover, a respective probe 104₁, 104₂, may be attached to an end of the mucin molecules 103₁, 103₂, especially to the end of the mucin molecules 103₁, 103₂ opposite to the detector surface 102, e.g., through a further respective linker molecule.

As such, the cluster of mucin molecules of the first type may facilitate multiple levels of intensity of the measured fluorescence or ORP, i.e., multiple intensity levels or multiple potential levels, which may allow for measuring the rate of protein degradation.

The device 500 further differs from the device 100 in that the device 500 may comprise a mucin molecule 503 of a second type, especially with known amino acid sequence and structure. As such, the mucin molecule 503 may be configured to be digested primarily by an enzyme of a second type produced in the mucus layer.

Furthermore, the mucin molecule 503 may be attached to the detector surface 102 through a further linker molecule 505, which may be similar or be the same as the linker molecules 105₁, 105₂ of the mucin molecule of the first type. Moreover, a further probe 504 may be attached to an end of the mucin molecule 503, especially to the end of the mucin molecule 503 opposite to the detector surface 102, e.g., through a further linker molecule.

The probe 504 of the mucin molecule 503 may correspond to the probes 104₁, 104₂ of the mucin molecules 103₁, 103₂. Alternatively, the probe 504 of the mucin molecule 503 and the probes 104₁, 104₂ of the mucin molecules 103₁, 103₂ may be different probes.

For example, the probe 504 of the mucin molecule 503 may be a fluorescent probe and the probes 104₁, 104₂ of the mucin molecules 103₁, 103₂ may be redox probes, or vice versa. Moreover, the device 500 may comprise additional mucin molecules of the second type, e.g., in a cluster or array, in an analogous manner to the cluster of the mucin molecules of the first type, especially to allow for measuring the rate of protein degradation due to the presence of different enzymes.

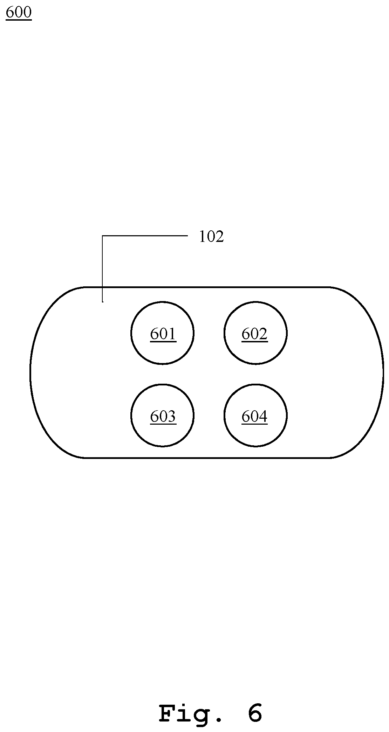

In Fig. 6, a third exemplary embodiment of the device 600 according to the first aspect of the invention is illustrated. In this regard, the device 600, especially the ingestible structure, is illustrated in a pill shape. For example, the device 600 may comprise, especially at the external surface 102, a first location 601, a second location 602, a third location 603, and a fourth location 604.

For example, a cluster of mucin molecules of a first type, such as the mucin molecules 103₁, 103₂ of Fig. 5, may be arranged in the first location 601. The mucin molecules of the first type may be configured to be digested primarily by an enzyme of a first type produced in the mucus layer.

In addition, a cluster of mucin molecules of a second type, such as the mucin molecules 103₁, 103₂ of Fig. 5, may be arranged in the second location 602. The mucin molecules of the second type may be configured to be digested primarily by an enzyme of a second type produced in the mucus layer.

Furthermore, a cluster of mucin molecules of a third type, such as the mucin molecules 103₁, 103₂ of Fig. 5, may be arranged in the third location 603. The mucin molecules of the third type may be configured to be digested primarily by an enzyme of a third type produced in the mucus layer.

Moreover, a cluster of mucin molecules of a fourth type, such as the mucin molecules 103₁, 103₂ of Fig. 5, may be arranged in the fourth location 603. The mucin molecules of the fourth type may be configured to be digested primarily by an enzyme of a fourth type produced in the mucus layer.

The detector 101 may be arranged inside of the ingestible structure such that the detector 101 may operably detect or measure the signals from the probes via the detector surface 102. In this regard, the detector 101 may simultaneously detect or measure the signals from each of the locations 601, 602, 603, 604. Alternatively, the detector 101 may sequentially detect or measure the signals from the locations 601, 602, 603, 604, e.g., in a time-multiplexed manner.

It should be understood that the number of locations illustrated here is exemplary and can be reduced to a smaller number or can be extended to a larger number.

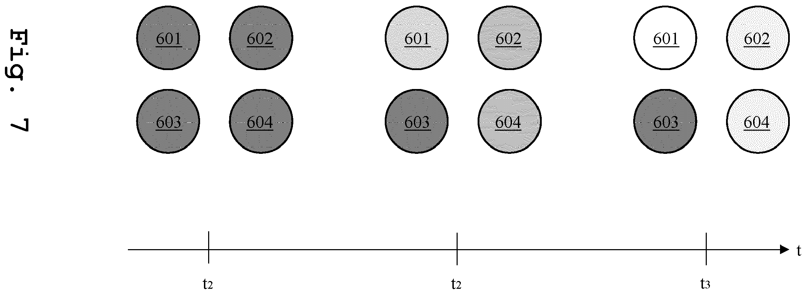

In Fig. 7, an exemplary enzymatic activity on the device 600 is illustrated as a function of time. As discussed before, the locations 601, 602, 603, 604 may indicate the type of glycodomains and the resulting intensity level of the respective locations 601, 602, 603, 604 may indicate the enzymatic activity corresponding to the type of glycodomains. In Fig. 7, the intensity level is illustrated in grayscale where higher intensity is shown in darker shades and lower intensity is shown in lighter shades.

For example, at the start at t₁, the intensity levels at the locations 601, 602, 603, 604 are all shown at maximum due to the absence of enzymatic digestion of the mucins. The intensity drops or reduces over time as the enzymes produced by the commensal bacteria may digest the O-glycans, thereby exposing the protein backbone to proteases. The proteases digest (degrade) the mucin molecule(s) resulting in the release, i.e. removal, of the probe, which leads to an alteration of the signal and/or intensity level of the signal. Thus, the mucin molecule status, in particular its degradation and/or removal, is detected by the detector.

Due to the presence of different enzymes at different locations within the mucus layer, e.g., within the GI-tract, the intensity at different locations 601, 602, 603, 604 may vary with respect to each other.

In this example, throughout the time instances t₂ and t₃, the intensity at the first location 601 is most reduced, the intensities at the second location 602 and the fourth location 604 are moderately reduced, and the intensity at the third location 603 is unchanged.

It can therefore be understood, for the third location 603, that the enzyme, which is able to digest this particular mucin, may not be present at all, e.g., throughout the whole GI-tract. Furthermore, the different intensity levels at different locations 601, 602, 603, 604 may correspond to the presence and activity of different glycodomain-specific enzymes, which may allow to assess or envisage the status of the mucus layer, e.g., the mucus layer lining the wall of the GI-tract.

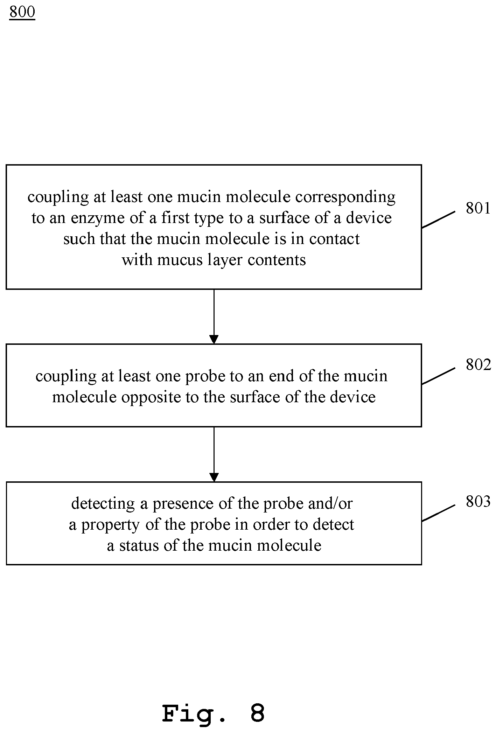

In Fig. 8, an exemplary embodiment of the method 800 according to the second aspect of the invention is illustrated. In a first step 801, at least one mucin molecule corresponding to an enzyme of a first type is coupled to a surface of a device such that the mucin molecule is in contact with mucus layer contents. In a second step 802, at least one probe is coupled to an end of the mucin molecule opposite to the surface of the device. In a third step 803, a presence of the probe and/or a property of the probe are detected in order to detect a status of the mucin molecule.

Therefore, the invention facilitates an in situ enzymatic activity detection scheme using mucin molecules attached to the external surface of the detector, which may measure the presence of enzymes produced by commensal bacteria residing in the mucus layer, e.g., in the mucus layer covering the cellular walls of GI-tract. The detector may continuously measure the degradation or removal of the mucin molecules due to digestion by the enzymes, e.g., the enzymes present inside the GI-tract.

Furthermore, by using multiple different mucin molecules attached to the external surface of the detector, a measurement on the presence and the activity of different enzymes can be provided, e.g., different enzymes produced by commensal bacteria residing inside the mucus layer of the GI-tract while the device may travel through the GI-tract. This may provide information on the health of mucus layer as well as a specific enzymatic signature, e.g., of the GI-tract.

It is important to note that, in the description as well as in the claims, the word "comprising" does not exclude other elements or steps and the indefinite article "a" or "an" does not exclude a plurality. A single element or other unit may fulfill the functions of several entities or items recited in the claims. Furthermore, the word "coupled" or "attached" implies that the elements may be directly connected together or may be coupled through one or more intervening elements. Moreover, the disclosure with regard to any of the aspects is also relevant with regard to the other aspects of the disclosure.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. A device (100, 500, 600) for detecting enzymatic activity in mucus layer, wherein the device comprises:
at least one mucin molecule (103) corresponding to an enzyme of a first type, wherein the mucin molecule (103) is coupled to a surface (102) of the device and is configured to be in contact with mucus layer contents,
at least one probe (104) coupled to an end of the mucin molecule (103) opposite to the surface (102) of the device, and
a detector (101) configured to detect a presence of the probe (104) and/or a property of the probe (104) in order to detect a status of the mucin molecule (103).

2. The device according to claim 1,
wherein the mucin molecule (103) comprises engineered human TR-O glycodomains decorated with O-glycans.

3. The device according to claim 1 or 2,
wherein the mucin molecule (103) is configured to be digested by the enzyme of the first type, especially produced by the commensal microbes that reside in the mucus layer.

4. The device according to any of claims 1 to 3,
wherein the probe (104) is an optical probe and the detector (101) is configured to detect an optical signal from the probe (104), preferably, wherein the optical probe is a fluorescent probe and the detector (101) is configured to detect a fluorescence of the fluorescent probe.

5. The device according to any of claims 1 to 3,
wherein the probe (104) is a redox probe and the detector (101) is configured to detect an oxidation-reduction activity of the redox probe.

6. The device according to any of claims 1 to 5,
wherein the device further comprises a linker molecule (105) configured to couple the mucin molecule (103) to the surface (102) of the device, the linker molecule (105) being inert to the mucus layer contents, especially to the enzymes produced by the commensal microbes that reside in the mucus layer, and inert to other gastrointestinal, GI, tract contents.

7. The device according to any of claims 1 to 6,
wherein the device further comprises:
at least one further mucin molecule (503) corresponding to an enzyme of a second type, wherein the further mucin molecule (503) is coupled to the surface (102) of the device and is configured to be in contact with the mucus layer contents, and
at least one further probe (504) coupled to an end of the further mucin molecule (503) opposite to the surface (102) of the device,
wherein the detector (101) is configured to detect a presence of the further probe (504) and/or a property of the further probe (504) in order to detect a status of the further mucin molecule (503).

8. The device according to claim 7,
wherein the further mucin molecule (503) is configured to be digested by the enzyme of the second type, especially produced by commensal microbes that reside in the mucus layer.

9. The device according to claim 7 or 8,
wherein the detector (101) is configured to detect the presence of the probe (104) and the presence of the further probe (504) simultaneously, or
wherein the detector (101) is configured to detect the presence of the probe (104) and the presence of the further probe (504) sequentially, and/or
wherein the detector (101) is configured to detect the property of the probe (104) and the property of the further probe (504) simultaneously, or
wherein the detector (101) is configured to detect the property of the probe (104) and the property of the further probe (504) sequentially.

10. The device according to any of claims 7 to 9,
wherein the mucin molecule (103) and the further mucin molecule (503) are coupled to the surface (102) of the device at predefined locations (601, 602, 603, 604), especially in an array formation.

11. The device according to any of claims 1 to 10,
wherein the status of the mucin molecule (103) or the further mucin molecule (503) is the enzymatic degradation and/or removal of the mucin molecule (103) or the further mucin molecule (503).

12. A method (800) for detecting enzymatic activity in mucus layer, the method comprises:
coupling (801) at least one mucin molecule corresponding to an enzyme of a first type to a surface of a device such that the mucin molecule is in contact with mucus layer contents,
coupling (802) at least one probe to an end of the mucin molecule opposite to the surface of the device, and
detecting (803) a presence of the probe and/or a property of the probe in order to detect a status of the mucin molecule.

13. The method according to claim 12,
wherein the method further comprises:
coupling at least one further mucin molecule corresponding to an enzyme of a second type to the surface of the device such that the further mucin molecule is in contact with the mucus layer contents,
coupling at least one further probe to an end of the further mucin molecule opposite to the surface of the device, and
detecting a presence of the further probe and/or a property of the further probe in order to detect a status of the further mucin molecule.

14. The method according to claim 12 or 13,
wherein the method further comprises:
detecting the presence of the probe and the presence of the further probe simultaneously, or
detecting the presence of the probe and the presence of the further probe sequentially, and/or
detecting the property of the probe and the property of the further probe simultaneously, or
detecting the property of the probe and the property of the further probe sequentially.

15. The method according to any of claims 12 to 14,
wherein the status of the mucin molecule or the further mucin molecule is the enzymatic degradation and/or removal of the mucin molecule or the further mucin molecule.
